(19) Europäisches Patentamt / European Patent Office / Office européen des brevets

(11) **EP 4 236 782 B1**

(12) **EUROPEAN PATENT SPECIFICATION**

(45) Date of publication and mention
of the grant of the patent:
**08.10.2025 Bulletin 2025/41**

(21) Application number: **21904165.4**

(22) Date of filing: **06.12.2021**

(51) International Patent Classification (IPC):
*A61B 5/053* (2021.01)      *A61B 5/0537* (2021.01)
*A61B 5/08* (2006.01)      *A61B 5/085* (2006.01)
*A61N 1/08* (2006.01)

(52) Cooperative Patent Classification (CPC):
**A61B 5/0536;** A61B 5/0537; A61B 5/085

(86) International application number:
**PCT/US2021/061939**

(87) International publication number:
**WO 2022/125409 (16.06.2022 Gazette 2022/24)**

(54) **TECHNIQUES FOR MODEL-BASED LUNG FLUID STATUS DETECTION**

VERFAHREN ZUR MODELLBASIERTEN LUNGENFLÜSSIGKEITSSTATUSDETEKTION

TECHNIQUES DE DÉTECTION D'ÉTAT DE FLUIDE PULMONAIRE BASÉE SUR UN MODÈLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.12.2020 US 202063124206 P**

(43) Date of publication of application:
**06.09.2023 Bulletin 2023/36**

(73) Proprietor: **Analog Devices, Inc.**
**Wilmington, MA 01887 (US)**

(72) Inventors:
• **SEO, Joohyun**
  **Wilmington, Massachusetts 01887 (US)**
• **AKL, Tony J.**
  **Wilmington, Massachusetts 01887 (US)**

(74) Representative: **Yang, Shu**
**Withers & Rogers LLP**
**2 London Bridge**
**London SE1 9RA (GB)**

(56) References cited:
EP-A1- 3 052 018        WO-A1-2018/104888
US-A1- 2005 124 908     US-A1- 2006 184 060
US-A1- 2014 276 166     US-A1- 2014 276 166
US-A1- 2014 358 021     US-A1- 2015 297 099
US-A1- 2016 135 741     US-A1- 2016 135 741
US-B1- 6 435 182

**EP 4 236 782 B1**

**Description**

RELATED APPLICATIONS

The present disclosure claims priority to U.S. Provisional Patent Application No. 63/124,206, entitled "MODEL-BASED LUNG FLUID STATUS DETECTION" and filed December 11, 2020.

FIELD OF THE DISCLOSURE

This disclosure relates generally to the field of lung fluid tomography and, more particularly, to techniques for model-based lung fluid status detection using multiple impedance measurements in combination with *a priori* knowledge of a region of interest.

EP3052018A1 discloses an electrical impedance tomography (EIT) system for determining electric properties of an internal body part of a patient comprising - an electrode array locatable in electrical contact with the body of a patient; device for applying an electrical current or voltage between two or more electrodes of the electrode array and for measuring generated electrical voltages and/or currents between other pair combinations of the electrode array; a Computing unit comprising a data processor and a storage unit, the storage unit comprising at least one reconstruction algorithm for use by the data processor for reconstructing the measured electrical voltages of the body part into electrical properties or changes of electrical properties, the data processor adapted to output a representation of the reconstructed electrical properties. According to the invention provision is made for the data processor being adapted to generate and/ or process a plurality of anatomical models descriptive of the body part, the System comprising an input Interface for inputting biometric data of the patient for use by the data processor, the data processor being adapted to select one of the models from the plurality of anatomical models on account of the biometric data of the patient, the data processor being adapted to use the selected model of the plurality of anatomical models for reconstructing the measured electrical voltages of the body part into electrical properties or changes of electrical properties.

US2014/358021A1 discloses a device and method for bioimpedance spectrography is corrected for breathing artefacts. A breathing signal is used in conjunction with the impedance signal to adjust for the time within the respiratory cycle at which the measurements are made. The correction allows the device to characterize tissue parameters accurately with fewer measurement points.

BRIEF DESCRIPTION OF THE DRAWINGS

To provide a more complete understanding of the present disclosure and features and advantages thereof, reference is made to the following description, taken in conjunction with the accompanying figures, wherein like reference numerals represent like parts, in which:

FIGURE 1 illustrates an example environment in which an illustrative system for model-based lung fluid status detection according to some embodiments of the disclosure;

FIGURE 2 is a block diagram illustrating exemplary functional components of the system of FIGURE 1, according to some embodiments of the disclosure;

FIGURE 3 illustrates operation of a 4-wire impedance measurement system in accordance with one embodiment;

FIGURE 4 illustrates an example electrical impedance tomography technique for lung fluid status detection in which multiple 4-wire impedance measurements using multiple (e.g., 8 or more) electrodes dispersed around a region of interest are performed to obtain a map of resistivity estimate without using any *a priori* knowledge of the region of interest;

FIGURE 5 illustrates a cross-sectional model of a human upper torso, showing various types of tissue (lung, heart, bone, and soft tissue) and air;

FIGURE 6 is a flowchart illustrating operation of a model-based fluid status detection technique using a single model of a region of interest;

FIGUREs 7A and 7B illustrates an example embodiment described herein for implementing a model-based lung fluid status detection system using multiple impedance measurements in combination with *a priori* knowledge of a region of interest;

FIGUREs 8A and 8B illustrates another example embodiment described herein for implementing a model-based lung fluid status detection system using multiple impedance measurements in combination with *a priori* knowledge of a region of interest; and

FIGURE 9 illustrates a schematic block diagram of a system for performing thoracic impedance measurements on a human subject.

DESCRIPTION OF EXAMPLE EMBODIMENTS OF THE DISCLOSURE

Lung resistivity is a physiological parameter that describes the electrical characteristics of the lungs. Lung composition changes due to changes in the lung tissues, fluid and air volume. Various diseases that can cause a change in lung composition may be monitored by measuring lung resistivity. Lung fluid status is one such change in lung composition that may be monitored by measuring lung resistivity.

In certain embodiments, a thoracic impedance (Z) measurement system is an inexpensive non-invasive system for assessing lung resistivity, and therefore lung fluid status, and is available in a wearable form-factor for enabling home use and monitoring. In some embodiments, the thoracic impedance measurement may be accomplished using four electrodes.

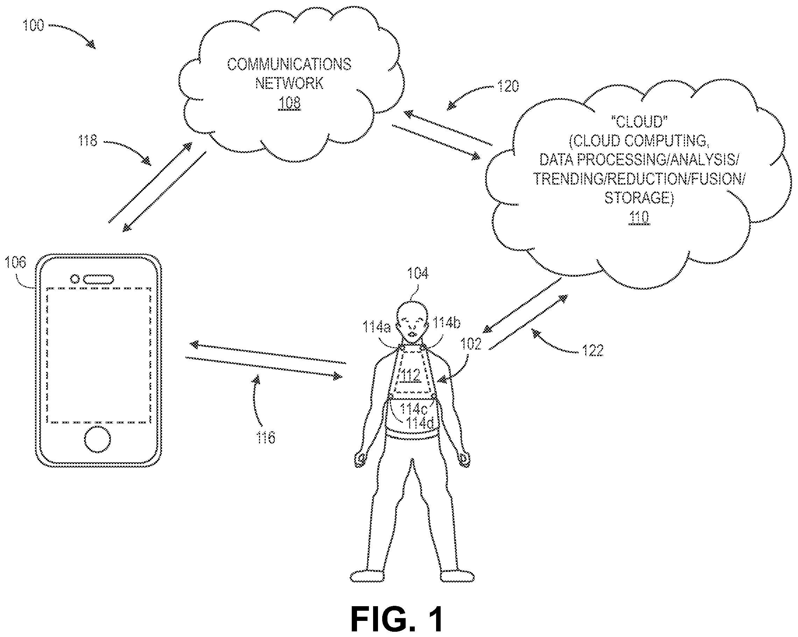

FIGURE 1 depicts an example environment 100 in which an illustrative embodiment of a system 102 for performing model-based lung fluid status detection in human subjects according to some embodiments of the disclosure. The monitoring may be performed in a continuous or periodic fashion. As shown in FIGURE 1, in accordance with one example embodiment, the system 102 includes a 4-wire thoracic impedance measurement module 112 and a plurality of surface electrodes/sensors 114a-114d (e.g., four (4) surface electrodes/sensors, or any other suitable number of surface electrodes/sensors). For example, one or more of the surface electrodes can be implemented as solid-gel surface electrodes, or any other suitable surface electrodes. The system 102 can be configured as a generally triangular-shaped device, or any other suitably shaped device, operative to make contact with one or more of the torso, upper chest, and neck areas, or any other suitable parts or areas of the body, of a human subject 104 via at least the plurality of surface electrodes/sensors 114a-114d.

In various implementations, the system 102 can have a configuration that allows it to be implemented within a wearable vest-like structure, as multiple patch-like devices, or any other suitable structure or device(s). In one possible environment, such as the environment 100, the system 102 may be operative to engage in bidirectional communications over wireless communication paths 116 with a smartphone 106, which, in turn, may be operative to engage in bidirectional communications over wireless communication paths 118 with a communications network 108 (e.g., the Internet). Alternatively, a direct link to the cloud 110 may be provided without requiring a hop through a base station or cell phone. The smartphone 106 is further operative, via the communications network 108, to engage in bidirectional communications over wireless communication paths 120 with the cloud 110, which can include resources for cloud computing, data processing, data analysis, data trending, data reduction, data fusion, data storage, and other functions. The system 102 is further operative to engage in bidirectional communications over wireless communication paths 122 directly with the cloud 110.

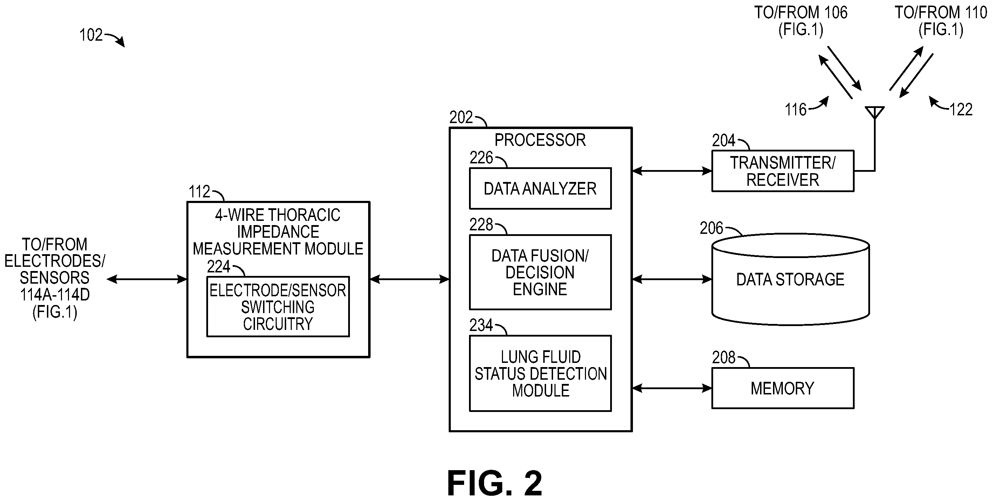

FIGURE 2 depicts an example block diagram of the system 102 for performing model-based lung fluid status detection in human subjects according to some embodiments of the disclosure. As shown in FIGURE 2, the system includes the thoracic impedance measurement module 112, a processor 202 and associated memory 208, a data storage 206 for storing thoracic impedance, lung resistivity, models, and related data, and a transmitter/receiver 204. The transmitter/receiver 204 can be configured to perform Bluetooth communications, Wi-Fi communications, or any other suitable short-range communications for communicating with the smartphone 106 (FIGURE 1) over the wireless communication paths 116. The transmitter/receiver 204 can be further configured to perform cellular communications or any other suitable long-range communications for communicating with the cloud 110 (FIGURE 1) over the wireless communication paths 122. In certain embodiments, the thoracic impedance measurement module 112 may further include electrode/sensor connection switching circuitry 224 for switchably making connections with the plurality of surface electrodes/sensors 114a-114d shown in FIGURE 1.

The processor 202 can include a plurality of processing modules such as a data analyzer 226 and a data fusion/decision engine 228. The transmitter/receiver 204 can include at least one antenna 210 operative to transmit/receive wireless signals such as Bluetooth or Wi-Fi signals over the wireless communications paths 116 to/from the smartphone 106, which can be a Bluetooth or Wi-Fi-enabled smartphone or any other suitable smartphone. The antenna 210 is further operative to transmit/receive wireless signals such as cellular signals over the wireless communications paths 122 to/from the cloud 110.

The processor 202 can further include a lung fluid status detection module 234 for performing model-based lung fluid status detection in human subjects in accordance with embodiments described herein and as described in greater detail below. It will be recognized that all or a portion of the processor 202, as well as modules shown as forming a part of the processor 202 (e.g., a portion or all of lung fluid status detection module 234), may additionally and/or alternatively be implemented in the cloud 110 (FIGURE 1) and may actually comprise multiple processors and/or processing elements for implementing the techniques described herein. It will be further recognized that other elements shown in FIGURE 2 as comprising part of the system 102 may be additionally and/or alternatively reside in the cloud 110 (FIGURE 1). In certain embodiments, the processor 202 may comprise a controller for controlling operations of measurement module/circuitry.

The transmitter/receiver 204 can include at least one antenna 210 operative to transmit/receive wireless signals such as Bluetooth or Wi-Fi signals over the wireless communications paths 116 to/from the smartphone 106, which can be a Bluetooth or Wi-Fi-enabled smartphone or any other suitable smartphone. The antenna 210 is further operative to transmit/receive wireless signals such as cellular signals over the wireless communications paths 122 to/from the cloud 110.

The operation of the system 102 for performing model-based lung fluid status detection in human subjects according to some embodiments will be further understood with reference to the following illustrative example, as well as FIGURES 1 and 2. In this illustrative example, at fixed times each day (e.g., two times per day) or continuously for a predetermined number of days while the human subject 104 is in a supine or upright position, the human subject or a human assistant positions the system 102 configured as the generally triangular-shaped device (or any other suitably shaped device) such that it makes contact with one or more of the subject's torso and upper chest and neck areas (or any other suitable parts or areas of the body) via the plurality of surface electrodes/sensors 114a-114d.

Having positioned the system 102 in contact with the human subject's torso and/or upper chest and/or neck areas, the 4-wire thoracic impedance measurement module 112 can be activated to gather, collect, sense, measure, or otherwise obtain thoracic impedance data from the human subject 104 and generate signals indicative thereof.

The 4-wire thoracic impedance measurement module 112 can perform thoracic impedance measurements using some or all of the plurality of surface electrodes 114a-114d that make contact with the skin of the human subject 104 on his or her torso, upper chest, and/or neck areas

In some embodiments, the thoracic impedance data from the thoracic impedance measurement module 112 may be provided to the data analyzer 226 for at least partial data analysis, data trending, and/or data reduction. In one embodiment, the thoracic impedance measurement data in combination with other metadata, such as medical history, demographic information, and other testing modalities, can also be analyzed, trended, and/or reduced "in the cloud" and made available in cloud-based data storage 110 with pre-set alerts for use in various levels of clinical interventions with respect to respiratory parameters.

The data analyzer 226 may provide the at least partially analyzed thoracic impedance data to the data fusion/decision engine 228, which may effectively at least partially fuse or combine the thoracic impedance data with other sensing data, in accordance with one or more algorithms and/or decision criteria, for subsequent use in making one or more inferences about the human subject 104. The processor 202 may then provide the at least partially combined thoracic impedance and other sensing data to the transmitter/receiver 204, which may transmit the combined thoracic impedance and sensing data either directly over the wireless communication paths 122 to the cloud 110, or over the wireless communication paths 116 to the smartphone 106. Next, the smartphone 106 can transmit, via the communications network 108, the combined thoracic impedance and sensing data over the wireless communication paths 118, 120 to the cloud 110, where it can be further analyzed, trended, reduced, and/or fused. It will be recognized that, as described above, communications data may be communicated directly to the cloud 110 without involvement of a smartphone/cell phone or base station.

The resulting curated combined sensing data can then be remotely downloaded by hospital clinicians for risk scoring/stratification, monitoring and/or tracking purposes.

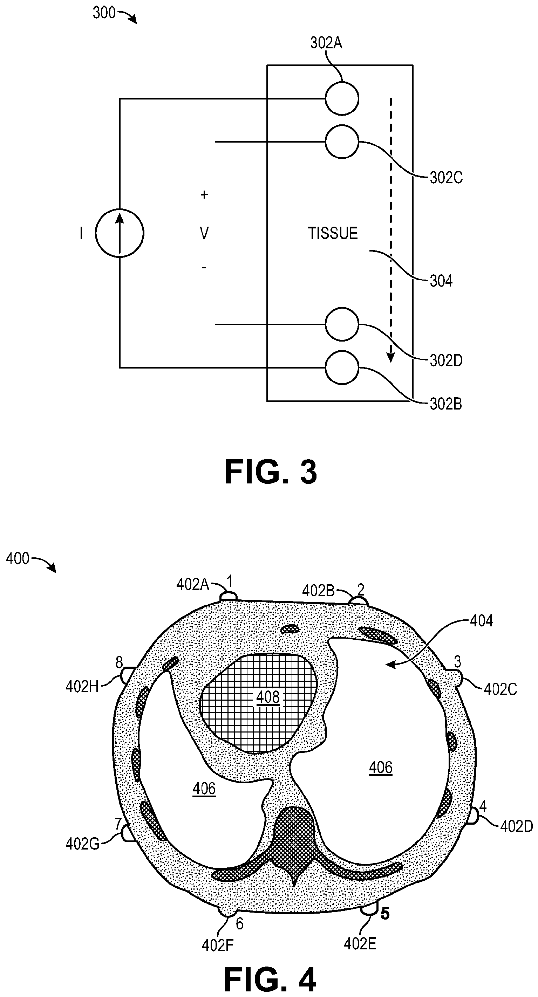

FIGURE 3 illustrates operation of a 4-wire thoracic impedance measurement system 300 in accordance with one embodiment. As shown in FIGURE 3, the system includes four electrodes 302A-302D. In operation, an alternating electrical current at a fixed excitation frequency I is injected from one of four electrodes (e.g., electrode 302A) to another one of the four electrodes (e.g., electrode 302B) through a region of interest 304 (e.g., a lung) and a voltage difference V across the remaining two electrodes (e.g., electrodes 302C and 302D) is measured. An impedance (Z) may be determined from those values (i.e., $Z = V/I$). A change in impedance indicates a change in the region of interest 304.

As will be described in greater detail, in accordance with features of embodiments described herein, the technique may also be performed using measurements at multiple excitation frequencies, with the final clinically useful information being derived from a combination of the multiple excitation frequency results, as opposed to the just the result from a single excitation frequency.

4-wire thoracic impedance measurement systems, such as the system 300, suffer from certain limitations. For example, when the region of interest 304 has high resistivity, such as is the case with a lung, the calculated impedance Z is not sensitive and specific to the resistivity change. Additionally, a small change in lung fluid status is easily obscured by other factors.

Referring now to FIGURE 4, illustrated therein is an electrical impedance tomography technique 400 for lung fluid status detection in which multiple 4-wire impedance measurements using multiple (e.g., 8 or more) electrodes 402A-402H dispersed around a region of interest 404 are performed to obtain a map of resistivity estimate without using any *a priori* knowledge of the region of interest. As shown in FIGURE 4, the region of interest 404 represents a human upper torso (or chest), including the individual's lungs 406 and heart 408.

In accordance with features of embodiments described herein, a model-based fluid status detection technique is performed using a limited number of (e.g., 5-6) electrodes in combination with *a priori* knowledge of the region of interest, or domain, with multiple 4-wire impedance measurements being performed to extract the domain information including the resistivity of the lung region ($\rho_{lung}$). Techniques described herein are more sensitive and specific to lung-fluid changes than a single 4-wire impedance measurement technique, while being less computationally heavy and complicated than traditional electrical impedance tomography techniques.

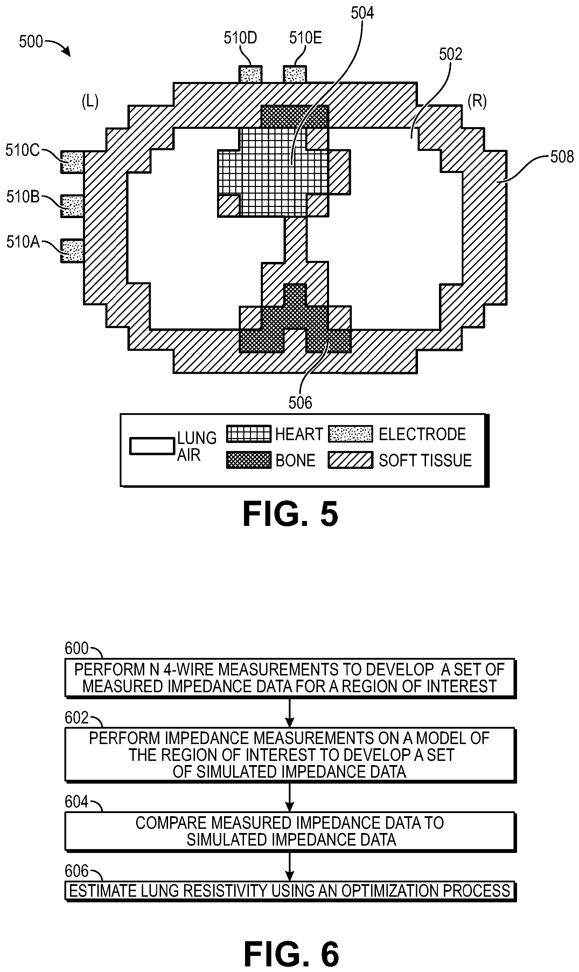

FIGURE 5 illustrates a cross-sectional model 500 of a human upper torso (or chest), showing various types of tissue, including lungs 502, heart 504, bone 506, and soft tissue 508. Lungs 502 are filled with air. Example placement of the electrodes 510A-510E is also shown in FIGURE 5. Lung resistivity (or lung fluid status) may be estimated within a single model, such as the model 500 shown in FIGURE 5.

FIGURE 6 is a flowchart illustrating operation of a model-based fluid status detection technique using a single model. In step 600, a number of (e.g., 5-6 (preferably fewer than 8)) electrodes are used to perform N 4-wire measurements to develop a set of measured impedance data $Z_{meas} = Z_1, Z_2, ... Z_N$ for a domain, or region, of interest. In step 602, simulated measurements are taken using a model of the domain of interest, such as the model 500 shown in FIGURE 5. Model parameters (x), typically consisting of continuous variables including $\rho_{lung}$, determine simulated impedance measurements $Z_{sim}(x) = [Z_{sim1}, Z_{sim2}, ... Z_{simN}]$. In step 604, a selected portion or all of the measured impedance data is compared to a selected portion or all of the simulated impedance data. In step 606, lung resistivity estimation is performed through an optimization process, in which a set of x, including $\rho_{lung}$, is identified that minimizes the cost function, for example, as described by but not limited to the following:

$$\min_{x} \ ||Z_{meas} - Z_{sim}(x)||^2$$

Solving one inverse problem for estimating lung resistivity, such as described above, presents a variety of challenges. For example, the measurement condition is highly likely to be non-negligibly different from what is assumed (e.g., electrode placement, lung size, tissue placement) in the simulation model. As a result, the final result may be overly sensitive to factors other than lung fluid status.

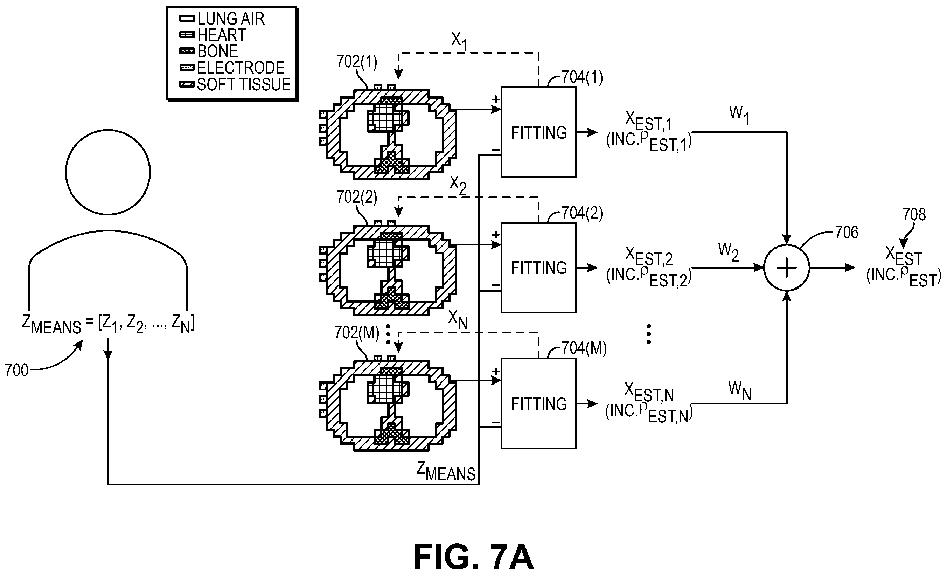

In accordance with features of embodiments described herein, and in order to ensure that the final result is sensitive and specific to lung fluid change and not to other factors, a multiple inverse problem is solved and the results are summarized based on the "fit" between the measured data and each problem. Referring now to FIGURE 7A, in one implementation, measured impedance data $Z_{meas}$ 700 of a region of interest (e.g., a human chest or upper torso) at a frequency $f$ is compared to simulated impedance data $Z_{sim}$ obtained from each of M sample models 702(1) - 702(M). Each of the M sample models 702(1)-702(M) represents a different possible combination of electrode placement and specific anatomical features (e.g., relative size and location of lung tissue, heart tissue, soft tissue, and bone) of the region of interest. A "fit" may be determined for each of the M models based on a comparison 704(1)-704(M) of the measured impedance data with the simulated impedance data from the model. Estimated model parameters for each of the models ($X_{est,1}$ - $X_{est,N}$), which include individual resistivity estimates ($\rho_{est,1}$ - $\rho_{est,N}$) for the models are integrated, or summarized 706, based on the "fit" between the measured data and the respective model to develop final model parameters ($X_{est}$), including a final resistivity estimate ($\rho_{est}$), 708 such that estimates from better "fitting" models are weighted more heavily in the final estimate than those from worse "fitting" models.

For example, the final resistivity estimate may be a weighted mean of the individual estimates, with the weight ($W_1$ - $W_N$) assigned to a model i, for example, defined by but not limited to:

$$1/f_{residual,cost,i}$$

where $f_{residual,cost}$ is the residual cost function value of the optimization for solving inverse problems. In the embodiment illustrated in FIGURE 7A, one measurement is understood as numerous possible placements and anatomical geometry with its own likelihood, or weight.

It will be noted that thoracic impedance measurements may vary depending on the excitation frequencies used to make the measurements, due to underlying changes how the electric current flows in the region of interest. For example, at a first excitation frequency, measurements may be more sensitive to changes in soft tissue, whereas at a different excitation frequency, measurements may be more sensitive to changes in lung tissue.

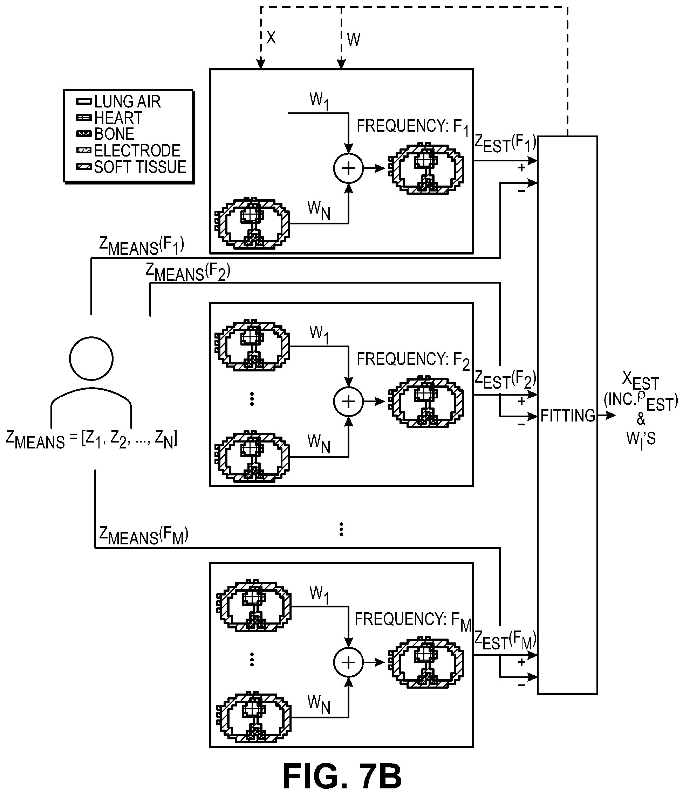

In accordance with aspects of embodiments described herein, as illustrated in FIGURE 7B, the techniques illustrated in FIGURE 7A may be applied to impedance data measured and estimated at each of multiple frequencies $f_1$ - $f_m$ to generate measured impedance data $Z_{meas}(f_1)$ - $Z_{meas}(f_m)$ and estimated (or simulated) impedance data $Z_{est}(f_1)$ - $Z_{est}(f_m)$. The results are combined by fitting $Z_{meas}(f_1)$ - $Z_{meas}(f_m)$ to respective simulated impedance measurements $Z_{est}(f_1)$ - $Z_{est}(f_m)$, thereby combining the data to develop final model parameters ($X_{est}$), including a final resistivity estimate ($\rho_{est}$) such that estimates from better "fitting" models are weighted more heavily in the final estimate than those from worse "fitting" models across the range of frequencies. The resulting parameters used to generate final clinical insights regarding changes in the region of interest .

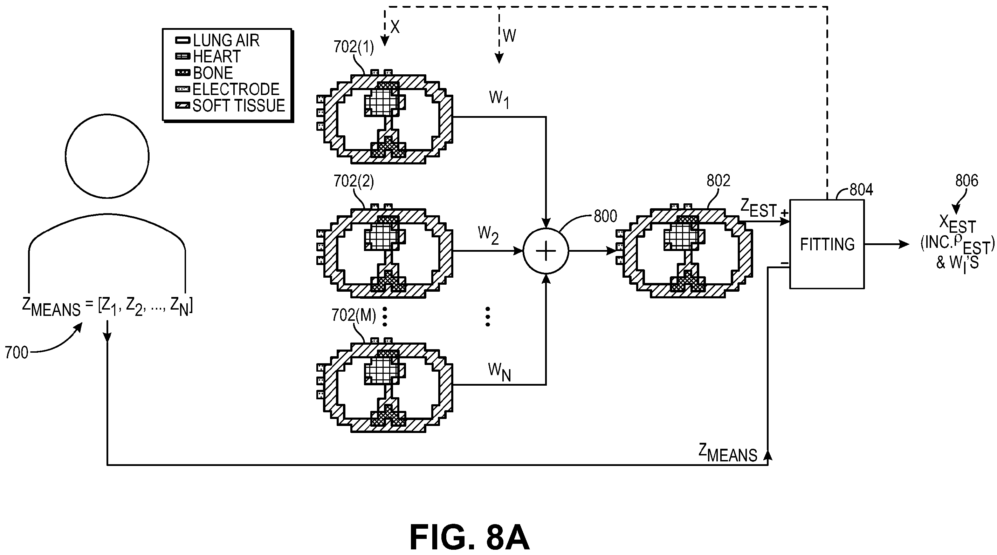

FIGURE 8A illustrates an alternative implementation in which the M models 702(1)-702(M) are "summarized" (via a mean of a weighted sum of the models) 800 into a single sample model 802 that is deemed to best represent the real measurement condition 700. The resulting single sample model 802 is used to produce estimated impedance data ($Z_{est}$) which is "fit" 804 to impedance data $Z_{meas}$ for producing final model parameters $X_{est}$, including a final resistivity estimate $\rho_{est}$, as well as the weights $W_i$ used in the weighted summation of the M sample models to produce the single sample model, 806.

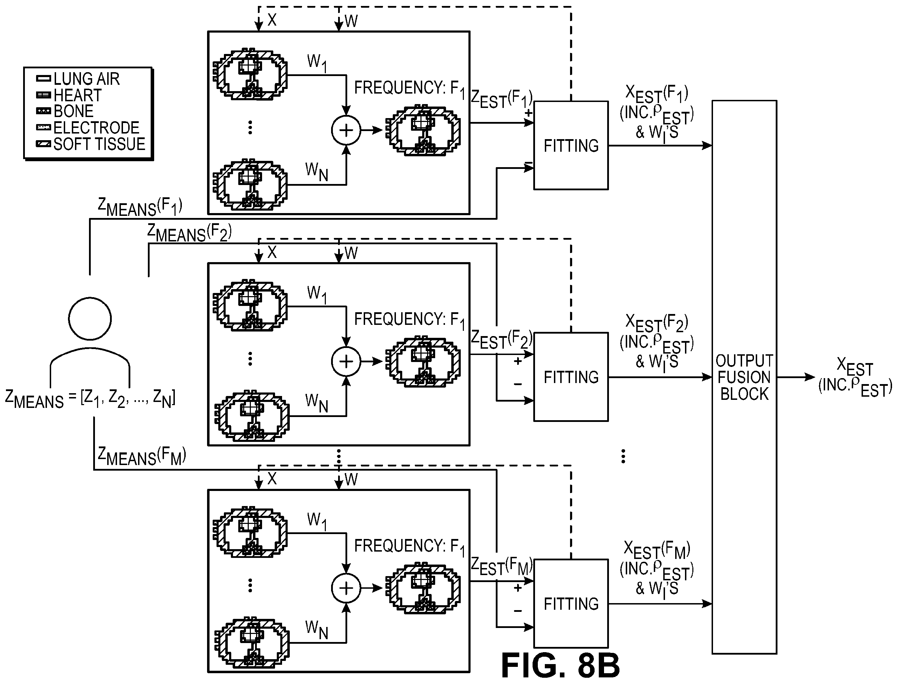

In accordance with aspects of embodiments described herein, as illustrated in FIGURE 8B, the techniques illustrated in FIGURE 8A may be executed for multiple excitation frequencies $f_1$ - $f_m$ to generate model parameters $X_{est}(f_1)$ - $X_{est}(f_m)$, including final resistivity estimates $\rho_{estf1}$ - $\rho_{estfm}$ as well as the weights $W_{if1}$ - $W_{ifm}$. The resulting parameters are combined and used to generate final clinical insights regarding changes in the region of interest.

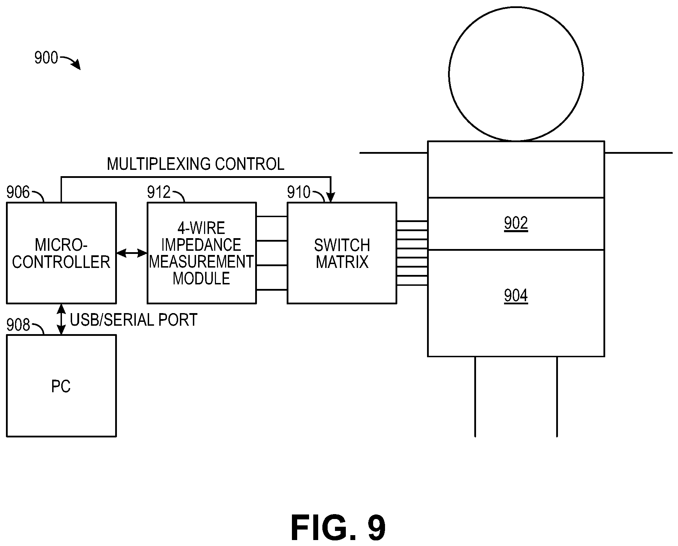

FIGURE 9 illustrates a block diagram of an example system 900 for obtaining measured impedance data $Z_{meas}$ of a region of interest (e.g., lung) of a patient as described above. As shown in FIGURE 9, an elastic chest band 902 having electrodes connected thereto is attached to the chest of a human subject 904. In the illustrated embodiment, three of the electrodes may be placed near the center of the chest of the subject 904 and three may be placed on the left side of the subject. A microcontroller unit (MCU) 906 under the control of a computer (PC) 908 controls the measurement sequence. In particular, the MCU 906 changes switch configuration 910 to perform different 4-wire impedance measurements using a 4-wire impedance measurement module 912 using the six (in the illustrated embodiment) electrodes placed on the chest of the subject 904.

It will be recognized that some or all of the processes, modules and/or devices illustrated in and described with reference to FIGURE 9 may be implemented using some or all of the processes, modules and/or devices shown in and described with reference to FIGURE 1 and/or FIGURE 2 and vice versa.

It should be noted that all of the specifications, dimensions, and relationships outlined herein (e.g., the number of elements, operations, steps, etc.) have only been offered for purposes of example and teaching only. Such information may be varied considerably without departing from the scope of the appended claims. The specifications apply only to one non-limiting example and, accordingly, they should be construed as such. In the foregoing description, exemplary embodiments have been described with reference to particular component arrangements. Various modifications and changes may be made to such embodiments without departing from the scope of the appended claims. The description and drawings are, accordingly, to be regarded in an illustrative rather than in a restrictive sense.

Note that with the numerous examples provided herein, interaction may be described in terms of two, three, four, or more electrical components. However, this has been done for purposes of clarity and example only. It should be appreciated that the system may be consolidated in any suitable manner. Along similar design alternatives, any of the illustrated components, modules, and elements of the FIGURES may be combined in various possible configurations, all of which are clearly within the broad scope of this Specification. In certain cases, it may be easier to describe one or more of the functionalities of a given set of flows by only referencing a limited number of electrical elements. It should be appreciated that the electrical circuits of the FIGURES and its teachings are readily scalable and may accommodate a large number of components, as well as more complicated/sophisticated arrangements and configurations. Accordingly, the examples provided should not limit the scope or inhibit the broad teachings of the electrical circuits as potentially applied to myriad other architectures.

It should also be noted that in this Specification, references to various features (e.g., elements, structures, modules, components, steps, operations, characteristics, etc.) included in "one embodiment", "exemplary embodiment", "an embodiment", "another embodiment", "some embodiments", "various embodiments", "other embodiments", "alternative embodiment", and the like are intended to mean that any such features are included in one or more embodiments of the present disclosure, but may or may not necessarily be combined in the same embodiments.

It should also be noted that the functions related to circuit architectures illustrate only some of the possible circuit architecture functions that may be executed by, or within, systems illustrated in the FIGURES. Some of these operations may be deleted or removed where appropriate, or these operations may be modified or changed considerably without departing from the scope of the present disclosure. In addition, the timing of these operations maybe altered considerably. The preceding operational flows have been offered for purposes of example and discussion. Substantial flexibility is provided by embodiments described herein in that any suitable arrangements, chronologies, configurations, and timing mechanisms may be provided without departing from the teachings of the present disclosure.

Numerous other changes, substitutions, variations, alterations, and modifications may be ascertained to one skilled in the art and it is intended that the present disclosure encompass all such changes, substitutions, variations, alterations, and modifications as falling within the scope of the appended claims.

Note that all optional features of the device and system described above may also be implemented with respect to the method or process described herein and specifics in the examples may be used anywhere in one or more embodiments.

The "means for" in these instances (above) may include (but is not limited to) using any suitable component discussed herein, along with any suitable software, circuitry, hub, computer code, logic, algorithms, hardware, controller, interface, link, bus, communication pathway, etc.

Note that with the example provided above, as well as numerous other examples provided herein, interaction may be described in terms of two, three, or four network elements. However, this has been done for purposes of clarity and example only. In certain cases, it may be easier to describe one or more of the functionalities of a given set of flows by only referencing a limited number of network elements. It should be appreciated that topologies illustrated in and described with reference to the accompanying FIGURES (and their teachings) are readily scalable and may accommodate a large number of components, as well as more complicated/sophisticated arrangements and configurations. Accordingly, the examples provided should not limit the scope or inhibit the broad teachings of the illustrated topologies as potentially applied to myriad other architectures.

It is also important to note that the steps in the preceding flow diagrams illustrate only some of the possible signaling scenarios and patterns that may be executed by, or within, communication systems shown in the FIGURES. Some of these steps may be deleted or removed where appropriate, or these steps may be modified or changed considerably without departing from the scope of the present disclosure. In addition, a number of these operations have been described as being executed concurrently with, or in parallel to, one or more additional operations. However, the timing of these operations may be altered considerably. The preceding operational flows have been offered for purposes of example and discussion. Substantial flexibility is provided by communication systems shown in the FIGURES in that any suitable arrangements, chronologies, configurations, and timing mechanisms may be provided without departing from the teachings of the present disclosure.

Although the present disclosure has been described in detail with reference to particular arrangements and configurations, these example configurations and arrangements may be changed significantly without departing from the scope of the present disclosure. For example, although the present disclosure has been described with reference to particular communication exchanges, embodiments described herein may be applicable to other architectures.

**Claims**

1. A method of detecting lung fluid status of a human subject, the method comprising:

   performing (600) multiple impedance measurements on a region of interest to obtain measured impedance data;
   comparing (604) the measured impedance data to simulated impedance data obtained from a plurality of models of the region of interest, wherein each of the models represents a different possible combination of electrode placement and a specific anatomical feature of the human subject;
   for each of the models, determining a fit of the model based on a comparison between the simulated impedance data obtained from the model and the measured impedance data; and
   integrating (606) individual resistivity estimates obtained from the models based on the fit of the model such that the individual resistivity estimate from a better fitting model is weighted more heavily in a final resistivity estimate than an individual resistivity estimate from a worse fitting model.

2. The method of claim 1, wherein the performing multiple impedance measurements comprises performing multiple 4-wire impedance measurements, and preferably wherein a maximum of eight electrodes are used to perform the multiple 4-wire impedance measurements.

3. The method of claim 1 or 2, wherein the specific anatomical features comprise at least one of a relative size and location of lung tissue, heart tissue, soft tissue, and bone.

4. The method of any of claims 1-3, wherein the final resistivity estimate is a weighted mean of the individual resistivity estimates, and preferably wherein a weight assigned to a particular model of the plurality of models is defined by an inverse value of a residual cost function value of an optimization for solving inverse problems, the residual cost function value corresponding to the particular model.

5. The method of any of claims 1-4, further comprising developing a single sample model using a weighted sum of the plurality of models based on a respective fit of the plurality of models.

6. The method of any of claims 1-5, wherein the multiple impedance measurements are performed at a single excitation frequency
   or
   wherein the multiple impedance measurements are performed at multiple excitation frequencies, and preferably further comprising executing the comparing, the determining, and the integrating for each of the excitation frequencies.

7. A system for detecting lung fluid status of a human subject, the system comprising:

   a plurality of electrodes on a chest of the human subject;
   a thoracic impedance detection module connected to the electrodes, the thoracic impedance detection module configured to perform the method of any of claims 1 to 6.

8. The system of claim 7, wherein the electrodes are connected to an elastic chest strap for attaching around a chest of the human subject to ensure correct location of the electrodes relative to the region of interest and/or wherein the plurality of electrodes further comprises three electrodes on the front of the chest and three of the electrodes on the left side of the chest.

9. A method of detecting lung fluid status of a human subject, the method comprising:

   performing (600) multiple impedance measurements on a region of interest to obtain measured impedance data;

generating a single sample model that represents the region of interest based on a plurality of models by determining a sum of a plurality of weighted models, wherein each of the plurality of models represents a different possible combination of electrode placement and a specific anatomical feature of the human subject;

generating simulated impedance data using the single sample model;

fitting (606) the simulated impedance data to the measured impedance data to produce weights applied to the plurality of models and a final resistivity estimate for the region of interest.

10. The method of claim 9, further comprising, prior to the generating, applying a weight to each of the plurality of models to produce the plurality of weighted models.

11. The method of claim 9 or 10, wherein the performing multiple impedance measurements comprises performing multiple 4-wire impedance measurements, and preferably wherein fewer than eight electrodes are used to perform the multiple 4-wire impedance measurements.

12. The method of any of claims 9-11, wherein the specific anatomical features comprise at least one of a relative size and location of lung tissue, heart tissue, soft tissue, and bone.

13. The method of any of claims 9-12, wherein the multiple impedance measurements are performed at a single excitation frequency or wherein the multiple impedance measurements are performed at multiple excitation frequencies, and preferably further comprising executing the generating and the fitting for each of the excitation frequencies.

14. A system for detecting lung fluid status of a human subject, the system comprising:

a plurality of electrodes on a chest of the human subject;

a thoracic impedance detection module connected to the electrodes, the thoracic impedance detection module configured to perform the method of any of claims 9 to 13.

15. The system of claim 14, wherein the electrodes are connected to an elastic chest strap for attaching around a chest of the human subject to ensure correct location of the electrodes relative to the region of interest and/or wherein the plurality of electrodes further comprises three electrodes on the front of the chest and three of the electrodes on the left side of the chest.

**Patentansprüche**

1. Verfahren zum Detektieren eines Lungenflüssigkeitsstatus eines menschlichen Subjekts, wobei das Verfahren umfasst:

Durchführen (600) mehrerer Impedanzmessungen an einer interessierenden Region, um gemessene Impedanzdaten zu erhalten;

Vergleichen (604) der gemessenen Impedanzdaten mit simulierten Impedanzdaten, die aus einer Vielzahl von Modellen der interessierenden Region erhalten wurden, wobei jedes der Modelle eine andere mögliche Kombination aus Elektrodenplatzierung und einem spezifischen anatomischen Merkmal des menschlichen Subjekts darstellt;

für jedes der Modelle Bestimmen einer Anpassung des Modells auf der Grundlage eines Vergleichs zwischen den simulierten Impedanzdaten, die aus dem Modell erhalten wurden, und den gemessenen Impedanzdaten; und

Integrieren (606) einzelner Widerstandsschätzungen, die aus den Modellen erhalten wurden, auf der Grundlage der Anpassung des Modells, so dass die einzelne Widerstandsschätzung aus einem besser passenden Modell in einer finalen Widerstandsschätzung stärker gewichtet wird als eine einzelne Widerstandsschätzung aus einem schlechter passenden Modell.

2. Verfahren nach Anspruch 1, wobei das Durchführen mehrerer Impedanzmessungen das Durchführen mehrerer 4-Draht-Impedanzmessungen umfasst, und vorzugsweise wobei maximal acht Elektroden verwendet werden, um die mehreren 4-Draht-Impedanzmessungen durchzuführen.

3. Verfahren nach Anspruch 1 oder 2, wobei die spezifischen anatomischen Merkmale mindestens eines von einer relativen Größe und Lage von Lungengewebe, Herzgewebe, Weichgewebe und Knochen umfassen.

4. Verfahren nach einem der Ansprüche 1-3, wobei die finale Widerstandsschätzung ein gewichteter Mittelwert der einzelnen Widerstandsschätzungen ist, und vorzugsweise wobei ein einem bestimmten Modell der Vielzahl von Modellen zugewiesenes Gewicht durch einen inversen Wert eines Restkostenfunktionswerts einer Optimierung zur Lösung inverser Probleme definiert ist, wobei der Restkostenfunktionswert dem bestimmten Modell entspricht.

5. Verfahren nach einem der Ansprüche 1-4, weiter umfassend das Entwickeln eines Einzelprobenmodells unter Verwendung einer gewichteten Summe der Vielzahl von Modellen auf der Grundlage einer jeweiligen Anpassung der Vielzahl von Modellen.

6. Verfahren nach einem der Ansprüche 1-5, wobei die mehreren Impedanzmessungen bei einer einzigen Erregungsfrequenz durchgeführt werden
oder
wobei die mehreren Impedanzmessungen bei mehreren Erregungsfrequenzen durchgeführt werden, und vorzugsweise weiter umfassend das Ausführen des Vergleichens, des Bestimmens und des Integrierens für jede der Erregungsfrequenzen.

7. System zum Detektieren eines Lungenflüssigkeitsstatus eines menschlichen Subjekts, wobei das System umfasst:

eine Vielzahl von Elektroden auf einer Brust des menschlichen Subjekts;
ein Thoraximpedanzdetektionsmodul, das mit den Elektroden verbunden ist, wobei das Thoraximpedanzdetektionsmodul so konfiguriert ist, dass es das Verfahren nach einem der Ansprüche 1 bis 6 durchführt.

8. System nach Anspruch 7, wobei die Elektroden mit einem elastischen Brustgurt verbunden sind, der um eine Brust des menschlichen Subjekts herum befestigt wird, um eine korrekte Lage der Elektroden relativ zu dem interessierenden Bereich sicherzustellen, und/oder wobei die Vielzahl von Elektroden weiter drei Elektroden auf der Vorderseite der Brust und drei der Elektroden auf der linken Seite der Brust umfassen.

9. Verfahren zum Detektieren eines Lungenflüssigkeitsstatus eines menschlichen Subjekts, wobei das Verfahren umfasst:

Durchführen (600) mehrerer Impedanzmessungen an einer interessierenden Region, um gemessene Impedanzdaten zu erhalten;
Erzeugen eines einzelnen Abtastmodells, das die interessierende Region darstellt, auf der Grundlage einer Vielzahl von Modellen, indem eine Summe einer Vielzahl von gewichteten Modellen bestimmt wird, wobei jedes der Vielzahl von Modellen eine andere mögliche Kombination aus Elektrodenplatzierung und einem spezifischen anatomischen Merkmal des menschlichen Subjekts darstellt;
Erzeugen simulierter Impedanzdaten unter Verwendung des einzelnen Abtastmodells;
Anpassen (606) der simulierten Impedanzdaten an die gemessenen Impedanzdaten, um Gewichte zu erzeugen, die auf die Vielzahl von Modellen angewendet werden, und eine finale Widerstandsschätzung für die interessierende Region.

10. Verfahren nach Anspruch 9, weiter umfassend, vor dem Erzeugen, das Anwenden eines Gewichts auf jedes der Vielzahl von Modellen, um die Vielzahl von gewichteten Modellen zu erzeugen.

11. Verfahren nach Anspruch 9 oder 10, wobei das Durchführen mehrerer Impedanzmessungen das Durchführen mehrerer 4-Draht-Impedanzmessungen umfasst, und vorzugsweise wobei weniger als acht Elektroden verwendet werden, um die mehreren 4-Draht-Impedanzmessungen durchzuführen.

12. Verfahren nach einem der Ansprüche 9-11, wobei die spezifischen anatomischen Merkmale mindestens eines von einer relativen Größe und Lage von Lungengewebe, Herzgewebe, Weichgewebe und Knochen umfassen.

13. Verfahren nach einem der Ansprüche 9-12, wobei die mehreren Impedanzmessungen bei einer einzigen Erregungsfrequenz durchgeführt werden
oder
wobei die mehreren Impedanzmessungen bei mehreren Erregungsfrequenzen durchgeführt werden, und vorzugsweise weiter umfassend das Ausführen des Erzeugens und des Anpassens für jede der Erregungsfrequenzen.

14. System zum Detektieren eines Lungenflüssigkeitsstatus eines menschlichen Subjekts, wobei das System umfasst:

eine Vielzahl von Elektroden auf einer Brust des menschlichen Subjekts;
ein Thoraximpedanzdetektionsmodul, das mit den Elektroden verbunden ist, wobei das Thoraximpedanzdetektionsmodul so konfiguriert ist, dass es das Verfahren nach einem der Ansprüche 9 bis 13 durchführt.

15. System nach Anspruch 14, wobei die Elektroden mit einem elastischen Brustgurt verbunden sind, der um eine Brust des menschlichen Subjekts herum befestigt wird, um eine korrekte Lage der Elektroden relativ zu dem interessierenden Bereich sicherzustellen, und/oder wobei die Vielzahl von Elektroden weiter drei Elektroden auf der Vorderseite der Brust und drei der Elektroden auf der linken Seite der Brust umfassen.

## Revendications

1. Procédé de détection d'état de fluide pulmonaire d'un sujet humain, le procédé comprenant :

    la mise en œuvre (600) de multiples mesurages d'impédance sur une région d'intérêt afin d'obtenir des données d'impédance mesurées ;
    la comparaison (604) des données d'impédance mesurées à des données d'impédance simulées obtenues à partir d'une pluralité de modèles de la région d'intérêt ; dans lequel chacun des modèles représente une combinaison possible différente de placement d'électrodes et d'une caractéristique anatomique spécifique du sujet humain ;
    pour chacun des modèles, la détermination d'un ajustement du modèle sur la base d'une comparaison entre les données d'impédance simulées obtenues à partir du modèle et les données d'impédance mesurées ; et
    l'intégration (606) d'estimations de résistivité individuelles obtenues à partir des modèles sur la base de l'ajustement du modèle, de sorte que l'estimation de résistivité individuelle provenant d'un modèle mieux ajusté soit pondérée plus lourdement dans une estimation de résistivité finale qu'une estimation de résistivité individuelle provenant d'un modèle moins bien ajusté.

2. Procédé selon la revendication 1, dans lequel la mise en œuvre de multiples mesurages d'impédance comprend la mise en œuvre de multiples mesurages d'impédance à 4 fils, et de préférence dans lequel un maximum de huit électrodes sont utilisées pour mettre en œuvre les multiples mesurages d'impédance à 4 fils.

3. Procédé selon la revendication 1 ou la revendication 2, dans lequel les caractéristiques anatomiques spécifiques comprennent au moins l'un ou l'une parmi une taille relative et un emplacement d'un tissu pulmonaire, d'un tissu cardiaque, d'un tissu mou, et d'un os.

4. Procédé selon l'une quelconque des revendications 1 à 3, dans lequel l'estimation de résistivité finale est une moyenne pondérée des estimations de résistivité individuelles, et de préférence dans lequel un poids attribué à un modèle particulier de la pluralité de modèles est défini par une valeur inverse d'une valeur de fonction de coût résiduel d'une optimisation pour la résolution de problèmes inverses, la valeur de fonction de coût résiduel correspondant au modèle particulier.

5. Procédé selon l'une quelconque des revendications 1 à 4, comprenant en outre le développement d'un modèle d'échantillon unique en utilisant une somme pondérée de la pluralité de modèles sur la base d'un ajustement respectif de la pluralité de modèles.

6. Procédé selon l'une quelconque des revendications 1 à 5, dans lequel les multiples mesurages d'impédance sont mis en œuvre à une fréquence d'excitation unique
ou
dans lequel les multiples mesurages d'impédance sont mis en œuvre à de multiples fréquences d'excitation, et de préférence comprenant en outre l'exécution de la comparaison, de la détermination, et de l'intégration pour chacune des fréquences d'excitation.

7. Système de détection d'état de fluide pulmonaire d'un sujet humain, le système comprenant :

    une pluralité d'électrodes sur une poitrine du sujet humain ;
    un module de détection d'impédance thoracique relié aux électrodes, le module de détection d'impédance thoracique étant configuré pour mettre en œuvre le procédé selon l'une quelconque des revendications 1 à 6.

8. Système selon la revendication 7, dans lequel les électrodes sont reliées à une sangle de poitrine élastique permettant un attachement autour d'une poitrine du sujet humain de manière à assurer un emplacement correct des électrodes par rapport à la région d'intérêt et/ou dans lequel la pluralité d'électrodes comprend en outre trois électrodes sur le devant de la poitrine et trois des électrodes sur le côté gauche de la poitrine.

9. Procédé de détection d'état de fluide pulmonaire d'un sujet humain, le procédé comprenant :

    la mise en œuvre (600) de multiples mesurages d'impédance sur une région d'intérêt afin d'obtenir des données d'impédance mesurées ;
    la génération d'un modèle d'échantillon unique qui représente la région d'intérêt sur la base d'une pluralité de modèles par la détermination d'une somme d'une pluralité de modèles pondérés, dans lequel chaque modèle de la pluralité de modèles représente une combinaison possible différente de placement d'électrodes et d'une caractéristique anatomique spécifique du sujet humain ;
    la génération de données d'impédance simulées en utilisant le modèle d'échantillon unique ;
    l'ajustement (606) des données d'impédance simulées aux données d'impédance mesurées de façon à produire des pondérations appliquées à la pluralité de modèles et une estimation de résistivité finale pour la région d'intérêt.

10. Procédé selon la revendication 9, comprenant en outre, avant la génération, l'application d'une pondération à chacun de la pluralité de modèles pour produire la pluralité de modèles pondérés.

11. Procédé selon la revendication 9 ou la revendication 10, dans lequel la mise en œuvre de multiples mesurages d'impédance comprend la mise en œuvre de multiples mesurages d'impédance à 4 fils, et de préférence dans lequel moins de huit électrodes sont utilisées pour mettre en œuvre les multiples mesurages d'impédance à 4 fils.

12. Procédé selon l'une quelconque des revendications 9 à 11, dans lequel les caractéristiques anatomiques spécifiques comprennent au moins l'un ou l'une parmi une taille relative et un emplacement d'un tissu pulmonaire, d'un tissu cardiaque, d'un tissu mou, et d'un os.

13. Procédé selon l'une quelconque des revendications 9 à 12, dans lequel les multiples mesurages d'impédance sont mis en œuvre à une fréquence d'excitation unique
ou
dans lequel les multiples mesurages d'impédance sont mis en œuvre à de multiples fréquences d'excitation, et de préférence comprenant en outre l'exécution de la génération et de l'ajustement pour chacune des fréquences d'excitation.

14. Système de détection d'état de fluide pulmonaire d'un sujet humain, le système comprenant :

une pluralité d'électrodes sur une poitrine du sujet humain ;
un module de détection d'impédance thoracique relié aux électrodes, le module de détection d'impédance thoracique étant configuré de façon à mettre en œuvre le procédé selon l'une quelconque des revendications 9 à 13.

15. Système selon la revendication 14, dans lequel les électrodes sont reliées à une sangle de poitrine élastique permettant un attachement autour d'une poitrine du sujet humain de manière à assurer un emplacement correct des électrodes par rapport à la région d'intérêt et/ou dans lequel la pluralité d'électrodes comprend en outre trois électrodes sur le devant de la poitrine et trois des électrodes sur le côté gauche de la poitrine.

**FIG. 1**

FIG. 2

FIG. 3

FIG. 4

**FIG. 5**

**FIG. 6**

FIG. 7A

FIG. 7B

FIG. 8A

FIG. 8B

EP 4 236 782 B1

900

MULTIPLEXING CONTROL

906
MICRO-
CONTROLLER

912
4-WIRE
IMPEDANCE
MEASUREMENT
MODULE

910
SWITCH
MATRIX

902

904

908
USB/SERIAL PORT

PC

# FIG. 9

**EP 4 236 782 B1**

**Patent documents cited in the description**

- US 63124206
- EP 3052018 A1
- US 2014358021 A1